# EUROPEAN PATENT APPLICATION

(11) **EP 4 282 854 A1**
(43) Date of publication of application: **29.11.2023**
(21) Application number: 22382503.5
(22) Date of filing: 25.05.2022
(51) Int. Cl.: C07C 213/06, C07C 219/06

(54) **PROCESS FOR THE PRODUCTION OF ESTERQUATS**

(71) Applicant: Unión Deriván, S.A., 08036 Barcelona (ES)
(72) Inventor: PERA SEIJO, Gorka, 50800 Zuera (ES)
(74) Representative: Sugrañes, S.L.P.

(57) **Abstract**

The present invention relates to a process for the production of esterquats characterized in that the quaternization step is performed using glycerol as the sole solvent. The invention also relates to the esterquat obtainable by such process and to a textile softener composition comprising it. The esterquat obtainable by the process of the invention is not inflammable and has improved softening properties.

## Description

### Technical field

The present invention relates to the field of cationic surfactants, in particular, to esterquats and, more particularly, it relates to novel process for the production of esterquats and to the esterquats produced therefrom, which have improved properties.

### Technical background

Esterquats, as is well-known in the art, is a term commonly used to designate a group of surface-active quaternary ammonium compounds, characterized by the fact that the hydrophobic chains are linked to the charged head group via ester bonds.

Due to the presence of ester groups in their structure, esterquats are more easily hydrolysable and biodegradable than, for example, alkyl quaternary surfactants ("alkylquats"), and that is one of the reasons of the wide use of these substances in many consumer products, in particular, in fabric softeners.

Esterquats are typically obtained by reaction of fatty acids with tertiary alkanolamines, to form an ester linkage between the carboxylic group of the fatty acid and the hydroxyl group of the alkanolamine, and subsequent quaternization. The esterification reaction is generally performed in the presence of a catalyst and at high temperatures to remove water and obtain high conversion. The quaternization is generally performed with alkyl halides or with dialkyl sulphates, in the presence of a polar solvent, such as 2-propanol (isopropyl alcohol, IPA).

Different esterquats can be obtained, depending on the structure of the alkanolamine and fatty acid starting materials, as well as depending on the stoichiometry of the reaction.

For example, esterquats prepared from triethanolamine are typically a mixture of quaternized mono-, di- and triesteramines, depending on the relative amount of fatty acid used in the esterification reaction. The quaternization of the esteramine mixture is generally carried out with dimethyl sulphate using IPA as solvent (Overkempe et al., Esterquats, in: Novel Surfactants. Preparation, applications and biodegradability; Holmberg K., Editor; Marcel Dekker, Second Edition, 2003; Surfactant Science Series Volume 114; Chapter 11, page 347-384).

The final esterquat product directly obtained after the quaternization reaction is generally used as cationic surfactant for the desired end use without any additional purification step, so the solvent used in the quaternization reaction, namely, IPA, typically remains in the final product, and may amount to about 5-15 % of the final esterquat mixture. The presence of isopropyl alcohol in the esterquat can be potentially problematic due to its inflammable nature. However, so far, it is considered indispensable for the preparation of esterquats, particularly of esterquats derived from triethanolamine, due to its optimal polarity and solvent properties.

The use of triglycerides, as an alternative to fatty acids for the preparation of esterquats, has been also disclosed in the art, so the esterification reaction with the tertiary alkanolamine is, in fact, a transesterification reaction. However, this alternative is generally seen as less attractive because the use of triglycerides leads to subproducts, particularly, mixtures of incompletely hydrolysed mono- and diglycerides and glycerine (Overkempe *et al., op. cit.*)*.*

In the US patent application US-A-2002/0002298 it is disclosed a process for the production of esterquats by transesterification of triglycerides with alkanolamines and subsequent quaternization in the presence of solvents, wherein the glycerol released during the transesterification needs to be continuously removed from the reaction equilibrium in order to reduce the proportion of undesired subproducts. Said glycerol removed during the transesterification can be then optionally used, in combination with isopropyl alcohol, as solvent for the subsequent quaternization reaction, with a ratio glycerol:IPA of about 1:2.5. The final mixture, therefore, has still a high proportion of IPA, and it also presumably contains some triglyceride-derived impurities.

Therefore, there is the need for an improved process for the preparation of esterquats which is suitable for obtaining esterquats of better properties, both in terms of their end-use applications and in terms of the safety of the product.

### Object of the invention

The object of the present invention is a process for the production of esterquats.

Another aspect of the invention is an esterquat obtainable by said process.

Another aspect of the invention is a textile softener composition comprising said esterquat.

### Detailed description of the invention

The object of the present invention is a process for the production of esterquats which comprises the following steps:
a) reacting a fatty acid with a tertiary alkanolamine; and
b) quaternizing the esteramine obtained in step a);
characterized in that the quaternization reaction b) is performed using glycerol as sole solvent.

The authors of the present invention have surprisingly found that glycerol can be used as the sole solvent for the quaternization reaction in the manufacture of esterquats, instead of isopropyl alcohol, and provides a final esterquat product which, not only is not inflammable, but has improved performance properties.

Along the present description, as well as in the claims, the singular forms "a," "an" and "the" are intended to include the plural forms as well, unless the context clearly indicates otherwise.

The terms "about" or "approximately" referred to amounts, as used herein, are meant to include the exact amount and also a certain deviation around the stated amount, namely of ±5%.

Unless stated otherwise, the percentages are meant to be by weight (wt%).

The numerical ranges disclosed herein are meant to include any number falling within the ranges and also the lower and upper limits.

### Step a): Reaction of a fatty acid with a tertiary alkanolamine

The first step for the preparation of esterquats consists of an esterification reaction, namely, the reaction between the carboxyl group in a fatty acid and the hydroxyl group in a tertiary alkanolamine, to provide an ester bond.

This esterification reaction can be carried out using common procedures known in the art of esterquat preparation, for example, as disclosed in the international patent application WO-A-91/01295, or in the European patent application EP-A-0295385, among others.

The term "tertiary alkanolamine", as is well-known in the art, means a substance of formula N(R₁)(R₂)(R₃), wherein at least one of R₁, R₂ and R₃ is a hydroxyalkyl group and the other are short alkyl chains, Typically, the hydroxyalkyl group has from 1 to 4 carbon atoms, preferably has 2 carbon atoms, and more preferably is 2-hydroxyethyl; and typically, the short alky chain has from 1 to 4 carbon atoms, and preferably is methyl or ethyl chains. It means, therefore, that either only one, or two or the three of R₁, R₂ and R₃ are hydroxyalkyl groups; in the latter case, therefore, no short alkyl chains are present. The hydroxyl group in the hydroxyalkyl moiety reacts with the carboxyl group in the fatty acid to form the ester bond.

Suitable examples of the tertiary alkanolamine to be used in the process of the present invention are triethanolamine (TEA) (i.e., R₁, R₂ and R₃ are all 2-hydroxyethyl groups: -CH₂-CH₂-OH), methyldiethanolamine (MDEA) (i.e. R₁ and R₂ are 2-hydroxyethyl groups and R₃ is methyl), dimethylethanolamine (DMEA) (i.e., R₁ is 2-hydroxyethyl and R₂ and R₃ are methyl groups), 3-(dimethylamino)-1,2-propanediol (DMAPD) (i.e., R₁ is 2,3-dihydroxypropyl: -CH₂-CH(OH)-CH₂-OH, and R₂ and R₃ are methyl groups), among others, and mixtures thereof. Also, suitable tertiary alkanolamines starting materials are the products of addition of the above described tertiary alkanolamines with ethylene oxide, typically with 1 to 10 mols of ethylene oxide.

In an embodiment, the tertiary alkanolamine is triethanolamine (TEA).

The above disclosed tertiary alkanolamines, are well-known substances which are readily available from commercial sources.

The fatty acid used for preparing the esterquat has a formula R-COOH, and has typically from 6 to 22 carbon atoms. The R alkyl chain in the fatty acid can be straight or branched, and can be unsaturated or saturated. In general, the R alkyl chain in the fatty acid may have 0, 1, 2 or 3 double bonds.

Some examples of suitable fatty acids are caproic acid (6:0), caprylic acid (8:0), 2-ethylhexanoic acid, capric acid (10:0), lauric acid (12:0), isotridecanoic acid, myristic acid (14:0), palmitic acid (16:0), palmitoleic acid (16:1 cis-9), heptadecanoic acid (17:0), stearic acid (18:0), isostearic acid, oleic acid (18:1 cis-9), elaidic acid (18:1 trans-9), petroselinic acid (18:1 cis-6), linoleic acid (18:2 cis-9,12), α-linolenic acid (18:3 cis-9,12,15), α- eleostearic acid (16:3 cis-9 trans-11,13), arachidic acid (20:0), gadoleic acid (20:1 cis-9), behenic acid (22:0), erucic acid (22:1 cis-13), or mixtures thereof.

The fatty acid is commonly obtained from natural sources, derived from fats and/or oils of animal or vegetal origin, and is typically a mixture of different fatty acids. Preferably, the fatty acid is a mixture of fatty acids having 12 to 18 carbon atoms, more preferably having 16 to 18 carbon atoms, derived from tallow, palm oil, sunflower oil, or coconut oil, among others, preferably from tallow or palm oil, preferably in hydrogenated or partially hydrogenated form, which are commercially available, for example, through the company Emery Oleochemicals, Croda or Union Deriván

Preferably, the fatty acid mixture has Iodine Value comprised between 30 and 70, preferably comprised between 40 and 55. As is well-known in the art, the Iodine Value is a measure of the unsaturation of fatty acids and is the amount of iodine in grams consumed by the reaction of the double bonds present in 100 g of fatty acid, determined by the method of ISO 3961.

The reaction between the fatty acid and the tertiary alkanolamine is preferably performed in absence of solvents at a temperature generally higher than 120° C, preferably higher than 150° C, for example comprised between 120° C and 220° C, preferably comprised between 150° C and 210° C, and more preferably comprised between 160° C and 200° C. The reaction is preferably performed under vacuum conditions, so the combination of high temperature and vacuum enhances the removal of water released from the esterification reaction. Typically, the pressure is kept under 1 bar, typically the pressure is from about 0.01 to 0.9 bar.

The mixture is allowed to react until substantially total conversion is achieved, which can be controlled by measuring the remainder acidity index in the reaction mixture, typically, by measuring the KOH consumption. In general, the reaction is considered to be complete when the acidity index is less than 5 mg of KOH per g of reaction mixture. The reaction time can range from about 1 hour to about 10 hours, and throughout this period the temperature is normally gradually raised until reaching the optimal reaction temperature, as stated above.

Preferably, a catalyst is added to accelerate the esterification reaction, which is typically an acidic catalyst, for example, an inorganic acid such as hypophosphorous acid or alkali metal salts thereof. Hypophosphorous acid, for example, can be used in amounts ranging from about 0.01 wt% to about 0.1 wt%, preferably ranging from 0.03 wt% and 0.07 wt%.

The reaction product of the esterification step a) is referred to herein as "esteramine", meaning that, besides the ester group newly formed by reaction of the carboxylic group in the fatty acid and the hydroxyl group in the alkanolamine, the reaction product obtained also comprises the amine group originally present in the alkanolamine.

The obtained esteramine reaction product is typically a complex mixture. Indeed, not only the fatty acid starting material is generally a mixture of different fatty acids, with different chain length and/or saturation, but also the degree of esterification achieved may not be complete, particularly for alkanolamine starting materials with more than one hydroxyl group. Thus, for example, for triethanolamine (TEA), a mixture of mono-, di- and tri-esteramines is typically obtained, of formulas (I), (II) and (III), respectively, as shown in Scheme 1 below, wherein R-COOH represents the fatty acid:

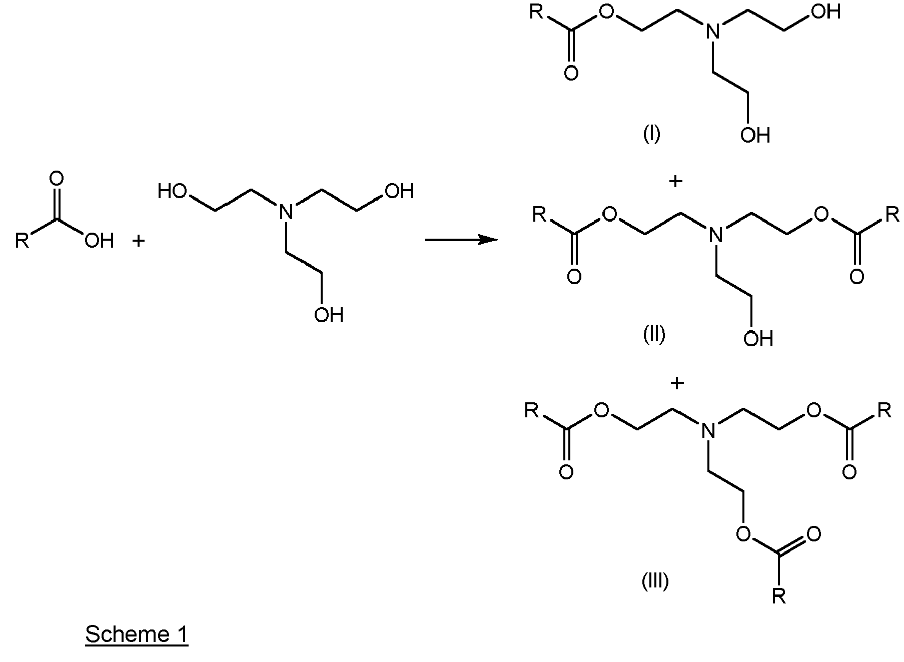

The relative amounts of the mono- and poly-ester reaction products depend on the ratio fatty acid:alkanolamine used. Furthermore, a remaining amount of unreacted tertiary alkanolamine may also be present in the reaction product mixture.

It follows that the composition of the final esterquat mixture, i.e., after the quaternization step b), is primary determined by the composition of the esteramine mixture, as discussed above.

The content of mono-, di- and tri-ester in the final esterquat mixture can be determined by HPLC, for example, as disclosed in the article Bahmaei et al., "Synthesis, IR, HPLC analysis and performances of palm fatty acids and triethanolamine-based esterquats", J. Surfact. Deterg., 2011, 14, 173-178.

In an embodiment, when triethanolamine is used as starting tertiary alkanolamine, the molar ratio fatty acid:alkanolamine is preferably comprised between 1.1:1 and 2.5:1, more preferably comprised between 1.2:1 and 2:1, still more preferably comprised between 1.3:1 and 1.8:1 and still more preferably comprised between 1.4:1 and 1.7:1.

### Step b): Quaternization

Quaternization reaction, as is well-known in the art, is the reaction between a tertiary amine (here, the esteramine or mixture of esteramines obtained in step a) of the present process) with an alkylating agent, to obtain a quaternary ammonium salt (here, the esterquat).

Thus, for example, the quaternization with dimethyl sulphate of the esteramine mixture obtained by esterification of TEA with fatty acid, as represented above in Scheme 1, gives rise to the following mixture of esterquats, as represented in Scheme 2:

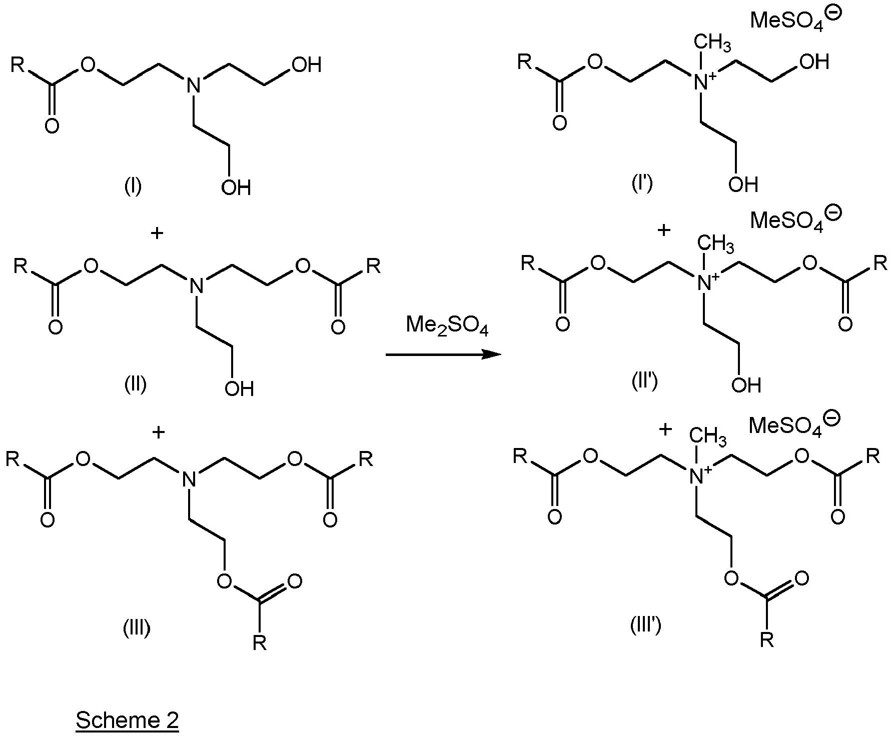

Although, theoretically, the quaternization reaction of the esteramine may be carried out in absence of solvent, a solvent is preferably added to the reaction mixture, as reported in the prior art (for example, in Overkempe et al., op. cit.).

The process of the present invention is characterized by the fact that the quaternization reaction is performed using glycerol as the sole solvent, instead of using isopropyl alcohol or isopropyl alcohol-containing solvent mixtures, as disclosed so far in the art.

The quaternization agent is typically an alkyl halide, a dialkyl sulphate or a dialkyl carbonate. For example, the quaternization agent is selected from dimethyl sulphate, diethyl sulphate, dimethyl carbonate, diethyl carbonate and methyl chloride.

Preferably, the quaternization agent is an alkyl halide or a dialkyl sulphate and is selected, for example, from dimethyl sulphate, diethyl sulphate and methyl chloride.

In a particular embodiment, the quaternization agent is dimethyl sulphate (DMS).

The amount of quaternization agent used is typically stochiometric or slightly sub-stoichiometric, i.e., a molar ratio quaternization agent:alkanolamine of from about 0.9:1 to about 1:1.

The quaternization reaction is typically performed at a temperature less than 100° C, for example, comprised between 50° C and 80° C, preferably comprised between 60° C and 70° C.

The reaction may be performed, for example, in the same reaction vessel where step a) has been performed, by first cooling the hot esterification reaction mixture until reaching the temperature suitable for the quaternization, then adding the glycerol, and subsequently slowly adding the quaternization agent, under stirring, while maintaining the temperature at the desired value during the addition. After the addition of the quaternization agent, the reaction mixture is allowed to proceed until completion.

The completeness of the reaction can be monitored, for example, measuring the amount of free unreacted tertiary amine. The amount of free unreacted amine can be measured, for example, by potentiometric titration with perchloric acid 0.1N with a Metrohm equipment, and a value of less than 0.1 meq/g is considered to show that the reaction is complete. Said titration can be carried out using methods known to the skilled person in the art, as disclosed in, for example, Gündüz et al., Analyst, 1988, 113(8), 1313-1316.

The amount of glycerol used in the quaternization reaction is typically such that it amounts to from about 5 wt% to about 15 wt%, relative to the total weight of the final reaction product mixture. Preferably the amount of glycerol is comprised between 6 wt% and 12 wt%, and more preferably comprised between 7 wt% and 10 wt%, relative to the total weight of the final product reaction mixture.

Optionally, after the quaternization reaction has been completed, a small amount of isopropyl alcohol (IPA) is added to the obtained esterquat, in order to further modulate the viscosity of the product. In case of adding IPA, the amount used is generally no more than 3 wt% relative to the total weight of the final mixture (including IPA), for example an amount comprised between 1 wt% and 3 wt% may be used.

Esterquat obtainable by the process of the invention and uses thereof

The esterquat obtained with the above-described process is, for the reasons discussed above, a complex mixture of substances, firstly due to the fact that the fatty acid starting material is itself a blend which can produce different combinations of esterified alkanolamines, and also due to the fact that different possible degrees of esterification and quaternization are possible. It follows, therefore, that an exact structural definition of the obtained esterquat is not possible.

Another aspect of the invention is, therefore, the esterquat obtainable by the process of the invention.

The esterquat of the invention is in the form of a viscose liquid which is ready for the desired end-use.

The esterquat of the invention, unlike most of the esterquats available in the prior art, is non inflammable, as it does not contain isopropyl alcohol or contains only small amounts of isopropyl alcohol, not superior to 3 wt%, while conventional esterquats generally comprise about 10-15 wt% of isopropyl alcohol and are, therefore inflammable. Being non inflammable is a big advantage as it allows for safer handling, storage and shipment of the esterquat.

Furthermore, surprisingly, it was found that the esterquat obtained with the new process, where isopropyl alcohol is replaced by glycerol as the solvent for quaternization, provided improved textile softening performance compared to conventionally obtained esterquats. Indeed, as discussed in the comparative assays of Example 4, in a blind test, the towels treated with the softener composition comprising the new esterquat of the present invention were chosen by all panellists as the softest, compared to towels treated with a softener composition comprising a standard esterquat, according to the prior art.

Moreover, as shown also in Example 4, the softener compositions prepared with the esterquat of the present invention have higher viscosity values than those prepared with the esterquats according to the prior art, for the same content of active ingredient. The higher viscosity of the softener is desirable, as, in general, it is favourably perceived by the users, as an indicator of high active matter content in the product.

Another aspect of the invention, therefore, is a textile softener composition comprising the esterquat of the invention.

The textile softener composition is prepared in a conventional way, typically, dispersing the esterquat in warm water, for example, at a temperature typically comprised between 25° C and 50° C, preferably comprised between 35° C and 45°C. Optionally, other formulation additives may be added. The softener composition comprises typically from 3 wt% to 40 wt% of the esterquat mixture of the invention, preferably from 5 wt% to 25 wt%.

Well-known formulation additives are, for example, perfumes, colorants, preservatives, salts such as calcium, sodium or magnesium chloride to adjust viscosity, hydrotropes, pH buffering agents, antifoaming agents, anticorrosion agents or antiredeposition agents, among others.

The present invention may be defined by the following embodiments:
1.- Process for the production of esterquats which comprises the following steps:
   a) reacting a fatty acid with a tertiary alkanolamine; and
   b) quaternizing the esteramine obtained in step a);
   characterized in that the quaternization reaction b) is performed using glycerol as sole solvent.
2.- Process according to embodiment 1, characterized in that the fatty acid has from 6 to 22 carbon atoms.
3.- Process according to embodiment 2, characterized in that the fatty acid has from 12 to 18 carbon atoms.
4.- Process according to any one of embodiments 1 to 3, characterized in that the fatty acid has iodine index value comprised between 30 and 70.
5.- Process according to embodiment 4, characterized in that the fatty acid has iodine index value comprised between 40 and 55.
6.- Process according to any one of embodiments 1 to 5, characterized in that the esterification reaction a) is performed at a temperature comprised between 120° C and 220° C, preferably comprised between 150° C and 210° C, and more preferably comprised between 160° C and 200° C.
7.- Process according to any one of embodiments 1 to 6, characterized in that the esterification reaction a) is performed under vacuum conditions at a pressure comprised between 0.01 bar and 0.9 bar.
8.- Process according to any one of embodiments 1 to 7, characterized in that a catalyst is added in step a).
9.- Process according to embodiment 8, characterized in that the catalyst is hypophosphorous acid or an alkali metal salt thereof.
10.- Process according to embodiment 9, characterized in that the catalyst is hypophosphorous acid, and is preferably is used in an amount comprised between 0.01 wt% and 0.1 wt%,
11.- Process according to any one of embodiments 1 to 10, characterized in that the alkanolamine in step a) is selected from the group consisting of triethanolamine (TEA), methyldiethanolamine (MDEA), dimethylethanolamine (DMEA), and 3-(dimethylamino)-1,2-propanediol (DMAPD).
12.- Process according to embodiment 11, characterized in that the alkanolamine is triethanolamine (TEA).
13.- Process according to embodiment 12, characterized in that the molar ratio fatty acid:TEA is comprised between 1.1:1 and 2.5:1, preferably comprised between 1.2:1 and 2:1, more preferably comprised between 1.3:1 and 1.8:1 and still more preferably comprised between 1.4:1 and 1.7:1.
14.- Process according to any one of embodiments 1 to 13, characterized in that the quaternization agent in step b) is selected from an alkyl halide, a dialkyl sulphate and a dialkyl carbonate, preferably is selected from dimethyl sulphate, diethyl sulphate, dimethyl carbonate, diethyl carbonate and methyl chloride, more preferably is selected from dimethyl sulphate, diethyl sulphate and methyl chloride.
15.- Process according to embodiment 14, characterized in that the quaternization agent is dimethyl sulphate.
16.- Process according to any one of embodiments 1 to 15, characterized in that the quaternization agent is used in an amount such that the molar ratio quaternization agent:alkanolamine is comprised between 0.9:1 and 1:1.
17.- Process according to any one of embodiments 1 to 16, characterized in that the quaternization reaction b) is performed at a temperature comprised between 50° C and 80° C, preferably comprised between 60° C and 70° C.
18.- Process according to any one of embodiments 1 to 17, characterized in that the amount of glycerol used in the quaternization reaction b) is such that it amounts to from 5 wt% to 15 wt%, preferably from 6 wt% to 12 wt%, and more preferably from 7 wt% to 10 wt%, relative to the total weight of the final product reaction mixture.
19.- Process according to any one of embodiments 1 to 18, characterized in that isopropyl alcohol is added to the reaction mixture after completion of the quaternization step b) in an amount comprised between 1 wt% and 3 wt%, relative to the total weight of the final product reaction mixture.
20.- Esterquat obtainable by the process according to any one of the embodiments 1 to 19.
21.- Textile softener composition comprising the esterquat of embodiment 20 dispersed in water.
22.- Textile softener composition according to embodiment 21, characterized in that it comprises between 3 wt% and 40 wt% of the esterquat, preferably between 5 wt% and 25 wt% of the esterquat.
23.- Textile softener composition according to embodiments 21 or 22, characterized in that it further comprises at least one additional component selected from perfumes; colorants; preservatives; salts such as calcium, sodium or magnesium chloride; hydrotropes; pH buffering agents; antifoaming agents; anticorrosion agents and antiredeposition agents.

### Examples

### Example 1.- Preparation of esterquat according to the invention

An esterquat according to the process of the present invention was prepared using the ingredients listed in the following table:

| **Ingredient** | **Amount (g)** |
|---|---|
| Fatty acid | 232 |
| TEA | 80 |
| Hypophosphorous acid | 0.15 |
| DMS | 63 |
| Glycerol | 33 |
| IPA | 11 |

### Esterification

The fatty acid used in the reaction was obtained from Union Deriván S.A. and was a fatty acid mixture comprising about the following chain distribution: C14 3%; C16 30%; C18 20% and C18' 38%, being the rest small amounts of C16', C17 and C18". The molar ratio fatty acid:TEA was calculated to be 1.55.

The fatty acids were first introduced into a reactor provided with heating means and heated to 80° C, TEA was then added and hypophosphorous acid was subsequently added. The mixture was progressively heated, during about 5 hours until reaching 170° C, and was maintained for 1 hour at this temperature, and for 2 additional hours at the same temperature under vacuum (pressure 300 mbar, approximately). After that, the esterification reaction was considered complete, as was confirmed by checking the acidity index, which was less than 5 mg KOH/g. The mixture was then cooled to about 65° C.

### Quaternization

Glycerol was added over the above obtained reaction mixture, maintaining the temperature at about 65° C. DMS was then gradually added, while still maintaining the temperature at about 65° C. Afterwards, the reaction was allowed to proceed at the same temperature for about 90 minutes, and, finally, IPA was added and the mixture was stirred for about 30 minutes. The reaction completeness was assessed by checking that free unquaternized amine, measured by potentiometric titration with perchloric acid, was below 0.1 meq/g.

The final esterquat obtained contained about 11 wt% of solvent (about 8 wt% glycerol and about 3 wt% IPA).

The esterquat mixture contained the following proportion of esters: 38 wt% of monoester, 52 wt% of diester and 10 wt% of triester.

### Example 2.- Preparation of additional esterquats according to the invention

In the esterquat prepared in Example 1, the molar ratio fatty acid:TEA was 1.55:1. Analogous procedure as disclosed in Example 1 was followed, but changing the molar ratio between the fatty acid and TEA, for the following esterquats:

| **Example** | **Molar ratio FA:TEA** |
|---|---|
| 2-1 | 1.77 |
| 2-2 | 1.83 |
| 2-3 | 1.93 |

### Comparative Example 1.- Preparation of esterquat according to the prior art

An esterquat was prepared according to the prior art, i.e., using IPA as solvent in the quaternization step. The following ingredients were used:

| **Ingredient** | **Amount (g)** |
|---|---|
| Fatty acid | 232 |
| TEA | 80 |
| Hypophosphorous acid | 0.15 |
| DMS | 63 |
| IPA | 44 |

The esterification step was performed in the same way as disclosed in Example 1.

### Quaternization

15 g of IPA were added over the esteramine reaction mixture obtained after the esterification step maintaining the temperature at about 65° C. DMS was then gradually added, while still maintaining the temperature at about 65° C. Afterwards, the reaction was allowed to proceed at the same temperature for about 90 minutes, and, finally, the rest of IPA was added and the mixture stirred for about 30 minutes. The reaction completeness was assessed by checking that free unquaternized amine, measured by potentiometric titration with perchloric acid, was below 0.1 meq/g.

### Example 3.- Softener composition with the esterquat of the invention

A 10 wt% softener composition was prepared dispersing 40 g of the esterquat obtained in Example 1 in 360 g of water. The preparation process was as follows: 200 g of water was heated at about 40° C, the esterquat heated at 50° C was added over the water and the mixture was stirred with a paddle stirrer at 200 rpm for about 15 minutes. 1 g of perfume was added and then the rest of water (160 g) at room temperature was added and the final mixture was further stirred for about 10 minutes at 200 rpm.

Analogously, a 7 wt% softener composition was prepared using suitable amounts of water and esterquat.

### Comparative Example 2.- Softener composition with the esterquat of the prior art

Comparative softener compositions of 10 wt% and 7 wt% concentration were prepared in the same way as disclosed in Example 3, but using the esterquat according to the prior art, prepared in the Comparative Example 1.

### Example 4.- Comparative assays of the softener composition of the invention vs. softener comprising esterquat of the prior art

### Softness evaluation

The 10 wt% softener composition of Example 3 and the analogous composition of Comparative Example 2 were tested for their softening effect on textiles. For that end, new non-used towels were treated with those two compositions in identical short programs (15 minutes) in a washing machine, and then allowed to dry.

A blind test was performed with a standard panel of testers, who were asked to assess the softness of two towels treated with either the softener of the invention or with the comparative softener and to choose which was the softest one. All panelist (100%) found that the towels treated with the softener composition of the invention were softer than the ones treated with the comparative.

Furthermore, the rewettability of the fabrics treated with both softener compositions was compared. The rewettability was assessed using methods available in prior art, such as those disclosed in, for example, Ginn et al., J. Am. Oil Chem. Soc., 1965, 42, 1084-1088 or in Mondal et al., J. Oleo Sci., 2016, 65, 663-670. It was found that fabrics treated with the softener composition according to the invention showed better rewettability than those treated with the softener composition according to the prior art.

### Viscosity

The viscosity of the softener compositions of Example 3 and Comparative Example 2 were measured using a viscosimeter Brookfield DV-1 at 20° C, spindle 1 at 12 rpm.

The viscosity values for the compared softener compositions are shown in the following table:

| | **Viscosity (centipoises)** | |
|---|---|---|
| *Concentration* | *Example3* | *Comparative Example 2* |
| 10 wt% | 350 | 270 |
| 7 wt% | 150 | 100 |

The softener compositions prepared with the new esterquats of the present invention had higher viscosity values than those prepared with esterquats according to the prior art.

### Example 5.- Stability studies

In stability studies at room temperature during 3 months, it was found that the softener compositions of the invention remained stable with about the same values of pH and viscosity at the end of the 3-month period.

## Claims

1. Process for the production of esterquats which comprises the following steps:
a) reacting a fatty acid with a tertiary alkanolamine; and
b) quaternizing the esteramine obtained in step a);
**characterized in that** the quaternization reaction b) is performed using glycerol as solvent.

2. Process according to claim 1, **characterized in that** the fatty acid has from 6 to 22 carbon atoms, preferably from 12 to 18 carbon atoms.

3. Process according to claims 1 or 2, **characterized in that** the esterification reaction a) is performed at a temperature comprised between 120° C and 220° C, preferably comprised between 150° C and 210° C, and more preferably comprised between 160° C and 200° C.

4. Process according to any one of claims 1 to 3, **characterized in that** a catalyst is added in step a), which is preferably hypophosphorous acid or an alkali metal salt thereof.

5. Process according to any one of claims 1 to 4, **characterized in that** the alkanolamine in step a) is selected from the group consisting of triethanolamine (TEA), methyldiethanolamine (MDEA), dimethylethanolamine (DMEA), and 3-(dimethylamino)-1,2-propanediol (DMAPD).

6. Process according to claim 5, **characterized in that** the alkanolamine is triethanolamine (TEA).

7. Process according to claim 6, **characterized in that** the molar ratio fatty acid:TEA is comprised between 1.1:1 and 2.5:1, preferably comprised between 1.2:1 and 2:1, more preferably comprised between 1.3:1 and 1.8:1 and still more preferably comprised between 1.4:1 and 1.7:1.

8. Process according to any one of claims 1 to 7, **characterized in that** the quaternization agent in step b) is selected from an alkyl halide, a dialkyl sulphate and a dialkyl carbonate, preferably is selected from dimethyl sulphate, diethyl sulphate, dimethyl carbonate, diethyl carbonate and methyl chloride, more preferably is selected from dimethyl sulphate, diethyl sulphate and methyl chloride.

9. Process according to claim 8, **characterized in that** the quaternization agent is dimethyl sulphate.

10. Process according to any one of claims 1 to 9, **characterized in that** the quaternization reaction b) is performed at a temperature comprised between 50° C and 80° C, preferably comprised between 60° C and 70° C.

11. Process according to any one of claims 1 to 10, **characterized in that** the amount of glycerol used in the quaternization reaction b) is such that it amounts to from 5 wt% to 15 wt%, preferably from 6 wt% to 12 wt%, and more preferably from 7 wt% to 10 wt%, relative to the total weight of the final product reaction mixture.

12. Process according to any one of claims 1 to 11, **characterized in that** isopropyl alcohol is added to the reaction mixture after completion of the quaternization step b) in an amount comprised between 1 wt% and 3 wt%, relative to the total weight of the final product reaction mixture.

13. Esterquat obtainable by the process according to any one of the claims 1 to 12.

14. Textile softener composition comprising the esterquat of claim 13 dispersed in water.

15. Textile softener composition according to claim 14, **characterized in that** it comprises between 3 wt% and 40 wt% of the esterquat, preferably between 5 wt% and 25 wt% of the esterquat.
